# EUROPEAN PATENT SPECIFICATION

(11) **EP 2 600 908 B1**
(45) Date of publication and mention of the grant of the patent: **21.05.2014**
(21) Application number: 11754542.6
(22) Date of filing: 02.08.2011
(51) Int. Cl.: A61L 15/22, A61L 15/44, A61L 15/58

(54) **BIOADHESIVE POLYMER-BASED CONTROLLED-RELEASE SYSTEMS, PRODUCTION PROCESS AND CLINICAL USES THEREOF**
BIOADHÄSIVE, POLYMERBASIERTE SYSTEM MIT KONTROLLIERTER FREISETZUNG, HERSTELLUNGSVERFAHREN UND KLINISCHE VERWENDUNGEN DAVON
SYSTÈMES À LIBÉRATION CONTRÔLÉE BASÉS SUR DES POLYMÈRES BIOADHÉSIFS, PROCÉDÉ DE PRODUCTION ET UTILISATIONS CLINIQUES DE CEUX-CI

(30) Priority: 02.08.2010 IT RM20100431
(43) Date of publication of application: 12.06.2013
(73) Proprietor: Quaglia, Fabiana, 80128 Napoli NA (IT); Marenzi, Gaetano, 80122 Napoli NA (IT); La Rotonda, Maria Immacolata, 80100 Napoli NA (IT); Sammartino, Gilberto, 80127 Napoli NA (IT); Miro, Agnese, 83027 Mugnano del Cardinale AV (IT); Ungaro, Francesca, 80127 Napoli NA (IT)
(72) Inventor: Quaglia, Fabiana, 80128 Napoli NA (IT); Marenzi, Gaetano, 80122 Napoli NA (IT); La Rotonda, Maria Immacolata, 80100 Napoli NA (IT); Sammartino, Gilberto, 80127 Napoli NA (IT); Miro, Agnese, 83027 Mugnano del Cardinale AV (IT); Ungaro, Francesca, 80127 Napoli NA (IT)
(74) Representative: Raimondi, Adriana
(86) International application number: PCT/IT2011/000282
(87) International publication number: WO 2012/017469

(56) References cited:
- WO-A1-01/13968
- SREENIVASAN K.: "On the Restriction of the Release of Water-Soluble Component from Polyvinyl Alcohol Film by Blending beta-Cyclodextrin", JOURNAL OF APPLIED POLYMER SCIENCE, vol. 65, no. 9, 7 December 1998 (1998-12-07), pages 1829-1832, XP002626193,
- SALMASO ET AL: "Cyclodextrin/PEG based hydrogels for multi-drug delivery", INTERNATIONAL JOURNAL OF PHARMACEUTICS, ELSEVIER BV, NL, vol. 345, no. 1-2, 13 November 2007 (2007-11-13), pages 42-50, XP022342759, ISSN: 0378-5173, DOI: DOI:10.1016/J.IJPHARM.2007.05.035
- VENTER J P ET AL: "Synthesis and evaluation of the mucoadhesivity of a CD-chitosan derivative", INTERNATIONAL JOURNAL OF PHARMACEUTICS, ELSEVIER BV, NL, vol. 313, no. 1-2, 26 April 2006 (2006-04-26), pages 36-42, XP025112907, ISSN: 0378-5173, DOI: DOI:10.1016/J.IJPHARM.2006.01.016 [retrieved on 2006-04-26]

## Description

### Field of the Invention

The present invention refers to release systems based on bioadhesive polymers, their production process and to their clinical uses in humans and animals. More in general, the uses are in the medical, pharmaceutical, cosmetic, cosmeceutical (cosmetic with therapeutic purposes) and nutraceutical fields.

### Prior Art

With the term "mucoadhesion" is meant the adhesion of a natural or synthetic polymer to a biological substrate. It is a practical method for the immobilization, targeting and controlled-release of drugs.

A mucoadhesive controlled release-device allows targeting a drug to a specific body site. The active principle may be charged onto a polymeric device which is capable of adhering to a mucous substrate and thus diffuse from the device directly into tissues.

Mucoadhesion also increases the nearness and the duration of contact between a polymer containing a drug and a mucous surface.

The term "mucus" is generally used to indicate the heterogeneous secretions which cover epithelial cells. Mucus is produced in different parts of the body, among which ears, eyes, nose, mouth and the gastrointestinal, reproductive and respiratory tracts. Its main role is to protect and lubricate the underlying epithelial tissue, in addition to act as a defence against pathogens.

With the term "bioadhesive systems" or "mucoadhesive systems", herein below used as synonyms, are meant those systems or medical devices based on polymers which constitute a support, also referred to as "platform" herein below, which assist in the adhesion to wet surfaces, as, for example, happens in particular epidermal or mucosal diseases, and which are able to exert a local or systemic effect in the human or animal body. An ideal bioadhesive system should adhere to wet surfaces for the time needed to completely release the drug therein contained.

The bioadhesive polymeric systems for the release of drugs are at the moment used, among other uses, in clinical applications for dental, orthopaedic, ophthalmologic and surgical purposes. Among the application sites are mouth, intestine, nose, eyes and vagina.

Bioadhesive systems are in general in the form of preparations for occlusive applications, gels, hydrogels, films or patches.

A bioadhesive system generally comprises one or more polymers which show a poor adhesive capacity at the dry state and become good adhesives in the hydrated state. Following contact with a wet or humid surface, such as, for example, a cutaneous surface with exudate or a mucosa, onto which is a watery hydrated film of mucin called "unstirred water layer"(UWL), absorption of water is seen by the polymer and, later, the interpenetration of the polymer chains within mucin.

The adhesion process of the polymer envisages formation of an intimate contact between the wound or mucous surface and the polymer chains of the bioadhesive system by means especially of mucous surface dehydration or of the cutaneous surface exposed, followed by formation of a network of secondary chemical bonds, in particular hydrogen bonds, between these two surfaces.

The mucoadhesion process allows the carried drug to be in tight contact with the action or absorption site for a prolonged time. This generates a better pharmacokinetic profile for the active principle and, as a consequence, a better efficacy of the therapy and possibly a reduction of side effects.

Mucoadhesive systems are prepared from polymers which are usually hydrophilic, natural or synthetic, which comprise groups that can interact with the mucous surface by means of such mechanisms as interpenetration, hydrogen bond formation, etc.

Mucoadhesive polymers can be classified as: not soluble mucoadhesives and soluble mucoadhesives. Not soluble mucoadhesives are materials which have a chemically crosslinked structure and which swells upon contact with an aqueous fluid. Their residence time on a mucosa depends from the mucus turnover and from cell shedding.

Mucoadhesive polymers, on the contrary, are polymers or copolymers, generally linear, which can be solubilized in water. Among the soluble mucoadhesive polymers currently are used synthetic polymers among which are derivatives of polyacrilic acids (cyanoacrilates, polyacrilates and polymetacrilates) polyethylene oxides or polyvinyl alcohols. Also, some semi-synthetic derivatives of cellulose (carboxymethyl cellulose and hydroxypropyl cellulose). Among the natural polymers are chitosan and a number of gums, among which are guar gum and xantan gum, hydrophilic derivatives of starch, alginates, pectin, and polysaccharides in general.

The cross-linking within a polymer system significantly influences chain motility and resistance to get dissolved. The crosslinked hydrophilic polymers swell in the presence of water while keeping their structure, while high molecular weight linear hydrophilic polymers swell and are easily dispersed in water. The bioadhesive capacity of a polymeric platform is in general influenced by the polymer chemical structure and from the polymer concentration as well as by pH of the surrounding environment. It is generally known that regarding polymers with linear chains, bioadhesive properties increase with an increase in the molecular weight and length of the polymer chains, while crosslinking modifies bioadhesive features, usually by making them worse. With the increase in the crosslinking density, chain mobility decreases and, thus, its effective length decreases, i.e., the one that can penetrate into the film of mucus, thus reducing the mucodhesive strength. The flexibility of the chain is critical for the interpenetration and interrelation with the gel of mucus. An enhanced chain mobility brings to an increased interdiffusion and interpenetration of the polymer into the mucous network.

The release devices based on mucoadhesive materials can be used for oral, rectal, eye, skin, transdermal and subcutaneous administration.

Drug release by the oral route is the most common method in the pharmaceutical field. With this administration type, mucoadhesive systems can release drugs in four main sites: mouth, stomach, small intestine and colon. In particular, release of the drug into the oral cavity finds versatile applications in the topical treatment of mouth disorders, such as dental diseases, stomatitis, viral and fungal infections, tumours of the oral cavity. The oral cavity can also be a useful site for the systemic administration of drugs, since the drugs absorbed by this route bypass the hepatic first pass metabolism.

Due to their flexibility and easy composition, the films based on mucoadhesive polymers are now a very interesting pharmaceutical form, in particular for the oral cavity pathologies. The local treatment for the oral cavity diseases actually seems to be very little effective in the case of conventional pharmaceutical forms due to the mobility of the tissues involved and of the continuous salivary flow which dilute and eliminates the drug, problems which, on the contrary, can be solved with an adequate bioadhesive formulation. In specific conditions it is also possible to obtain a transmucosal absorption of the active principle and, thus, to determine a systemic effect.

The fundamental steps which allow a drug to concentrate in a specific area of the mucosa or of the wet cutis consist of an hydration phase of the mucoadhesive system following the contact with the mucosa/saliva/exudate, followed by dissolution of the active principle in the mucoadhesive system and finally by partition in the mucosa. This implies that the step of incorporating a drug into the film is of paramount importance for concentrating it into the mucosa which is in close contact with the mucoadhesive system in reasonably short periods, shorter than the degradation time of the matrix which is soluble in the salivary fluid, and to enhance the local bioavailablility and the therapeutic effect.

Such steps are particularly critical in the case of lipophilic drugs, which, in spite of the fact that they have a high mucosal permeability, show a slow rate of dissolution in the hydrated bioadhesive system and, as a consequence, a poor partition into the mucosa.

Bioadhesive films may be prepared by means of different methods and a drug can be therein incorporated by different ways. The most widely used method, called film casting, involves solubilizing the polymer and the drug in a solvent or mixture of solvents (generally water or a mixture of organic solvent/water) which is then cast at the desired thickness onto a support (backing) where evaporation of the solvent and film solidification happen.

A methodological issue refers to the integration of drugs which are poorly soluble in water into bioadhesive systems, which in general are hydrophilic. This step is a critical one for most of the drugs which are poorly soluble in aqueous systems and which may be found in the film in their crystal form. In such a case, the drug gets distributed in a non-homogeneous way in the film, later on showing problems when the initial film must be divided in smaller portions, since conformity to quality control can not be guaranteed in terms of dosage uniformity (European Pharmacopeia 7th Ed.). A film containing a poorly soluble drug in crystallized form is the origin of further issues, since, once applied to a mucosa or onto a wet cutis or onto a wound, the drug gets solubilised into the UWL with excessively slow kinetics, which translates into a lower drug concentration in the damaged tissue with respect to the desired one.

Among the bioadhesive polymers, polyethylene oxide (PEO) is a hydrophilic and hydrosoluble polymer already approved for biomedical applications and available in a wide range of molecular weights.

The high molecular weight PEO is particularly useful for making bioadhesive supports (or platforms). The PEO can be compressed to make hydrophilic prolonged-release matrixes, can be used to make viscous solutions and can be processed as a thin and flexible film. Lipophilic films can be incorporated in the PEO platforms by compression of previously mixed solids, or, in case of systems which entail a step of PEO solubilization, in a co-solvent. Independent of the preparation procedure, lipophilic drugs are, anyway, present as discrete crystals inside the platform, which entails a series of issues, as already discussed above, among which there is poor tissue availability.

The simplest strategy to face these issues provides the use of volatile organic solvents capable of co-solubilizing the drug and the polymer.

Anyway, a possible alternative involves acting on the apparent solubility of the drug in water, enhancing it thanks to the help of complexant excipients such as cyclodextrins (also indicated as CD herein below). Inclusion drug/cyclodextrin complexes to be inserted in the PEO are known, which actually can interact with the cyclodextrins to make said complexes.

Cyclodextrins (CD) are cyclic oligosaccharides obtained by hydrolysis and enzymatic cyclization of starch and are made of glucopyranoside units linked by alpha-1,4 bonds, and, based on the number of units, they are classified as alpha-cyclodextrins (alpha-CD, aCD, αCD), made of six monosaceharide units, beta-cyclodextrins (beta-CD, bCD, β-CD) with seven units and gamma-cyclodextrins with eight units.

Cyclodextrins can form solid inclusion complexes (so called host guest complexes) with non polar substances, or portions thereof, by means of a molecular complexing mechanism. In these complexes the guest molecules place themselves inside the lipophilic groove of the cyclodextrin forming a complex characterized by a complexation constant that is a function of the CD affinity for the guest. Thanks to this property of their own, CDs are able to enhance the water apparent solubility of an active principle, its dissolution rate, its stability and bioavailability.

Currently used cyclodextrins are preferably semi-synthetic derivatives of β-cyclodextrin, soluble in highly polar organic solvents, marketed by Roquette or Wacker such as hydroxypropyl-βCD (HPβCD) and Methyl-βCD. The HPβCD marketed by Roquette under the name Kleptose^{®} HP is an amorphous white powder, with a solubility in water higher than 1g/ml. Further to this, it has a good stability both in acidic and in basic milieu and can be used to administrate drugs both by injection and by the oral route. The second one, under the name Kleptose^{®} Crysmeb^{®} exp, is a mixture of cyclodextrins which contain from one to seven methyl groups. Crysmeb^{®} has an average of 4 methyl groups for one cyclodextrin molecule and has an average molecular weight of 1191 dalton. It is a white powder, easily soluble in water and its solubility grows with the temperature, and, in addition, it is very stable both in and acidic and in basic milieus.

Different semi-synthetic CD derivatives, considered as GRAS excipients (Generally Regarded As Safe) are available and are widely used in the pharmaceutical field. Among them HPβCD appears extremely versatile thanks to its capacity of also being soluble in organic solvents.

The patent application US2007/0292479 discloses ternary mixtures of a hydrosoluble polymer such as polyvinyl alcohol in combination with HPβCD with the aim of producing films capable to carry steroidal hormones by the systemic route.

The process disclosed in Patent Application US2007/0292479 has the aim of avoiding the use of organic solvents to solubilize a drug by providing a preliminary solubilization of the drug in an aqueous concentrate solution of HPβCD. This process has the disadvantage of working only and exclusively if the drug is completely soluble in the starting aqueous solution. Unfortunately, not all of the drugs are soluble in concentrate aqueous solutions of HPβCD due to the fact that they interact with the CD in different measure, which, sometimes, is limited. From this it ensues that the poorly soluble and bioavailable particles of drugs are embedded into the film. The film can also be made from very concentrate polymer solutions, a thing that evidently constitute a disadvantage form the industrial point of view due to the need to mix extremely viscous solutions dissipating energy and due to the fact that such solutions easily incorporate air. In said process, formation of drug/HPβCD in solution, which is thermodinamically favored, can later render disadvantageous the incorporation of HPβCD into the polymer.

Herein below, the different cyclodextrins will be generically indicated with the acronym CD.

Bioadhesive polymers are known which contain CDs [Sreenivasan K. J. of Applied Polymer Science vol. 65, n. 9 (1998-12-07) 1829-1832; Salmaso et al., Int. J. of Pharmaceuticals, Elsevier BV, NL, vol. 345, n. 1-2 (2007-11-13) 42-50; Venter J.P. et al., Int. J. of Pharmaceuticals, Elsevier BV, NL, vol. 313, n. 1-2 (2006-04-26) 36-42], anyway the reaction conditions described make use of the CDs as crosslinkers of the polymer, and the resulting product, more crosslinked with respect to the starting polymer, shows modified mucoadhesion properties which ensues a slower release of the drug therein contained (as reported by the paper).

Sreenivasan and Salmaso describe the chemical synthesis of hydrogels containing CDs embedded into the structure. In Sreenivasan the PVA and bCD system is crosslinked by glutaric aldehyde; in Salmaso, the CD works as the chemical crosslinker for PEG. The crosslinked PEG does not show bioadhesive properties, unless in the hydrogel pendant PEG groups are left [Engineering design and molecular dynamics of mucoadhesive drug delivery systems as targeting agents. Serra L, Doménech J, Peppas NA. Eur J Pharm Biopharm. 2009 Mar;71(3):519-28.], a thing that does not happen in the hydrogel of Salmaso, since a diamino-PEG is used. Therefore, the CD of the prior art is not released by the hydrogel, but is trapped within the hydrogel itself. Furthermore, the hydrogels described release a drug with a prolonged timing (hours) and, by raising the CD content, the release becomes longer. Further to this, the system described in Salmaso shows to highly absorb water (i.e., a high swelling) which may be disadvantageous for mucoadhesion. Actually, even though hydration is essential to the relaxation and interpenetration of the polymer chains, an excess hydration may bring to a reduction of mucoadhesion and/or to retention due to the formation of a viscous mucilage.

In Venter a chemical derivative is prepared starting from chitosan and CD. The new polymer thus obtained shows a lower mucoadhesion with respect to the starting polymer, bringing to evidence that the chemical modification introduced by adding CD is disadvantageous for the interaction with mucus.

Also, processes for the preparation of polymer with CDs are known (patent application WO01/13968 and EP1449525) which are based on a dry mixing of components and compression at high temperature, which have the disadvantage to generate a lack in homogeneity of the CD distribution in the final product.

In particular, in WO01/13968 an adhesive sensitive to pressure is disclosed which is prepared by fusion and in which hydrocolloids and CDs are dispersed (temperatures >90°C). The manufacturing process does not envisage the use of a liquid vehicle, which is instead thought to be essential to favour the interaction between polymer, CDs and drugs. In the systems thus obtained, the CD does not get intimately mixed with the polymer by creating molecular interactions and remains as a separate and discontinuous phase.

It is evident from these considerations that the development of new strategies for the formulation of release systems, in particular in the form of mucosal films capable of topically carrying drugs which are poorly soluble in water, is prone to a relevant innovation from the technological and formulation point of view.

In particular, the need for obtaining a system for a controlled release based on bioadhesive polymers is felt, which might enable an effective carrying of active principles which are particularly difficult to solubilize in biological fluids, keeping the mucoadhesive properties of the polymer(s) used, and enhancing at the same time the local bioavailability of the active principle by means of the use of CDs.

It is also felt the need for keeping constant the release properties of products made from ternary mixtures polymer/CD/active principles throughout the extension of said products.

### Summary of the Invention

It is an object of the present invention a system for a controlled release based on bioadhesive polymers comprising a biodhesive polymer or copolymer and cyclodextrins, wherein the added cyclodextrins are mixed with the polymer in a homogeneous manner so that its bioadhesive features are not substantially modified. To this mixture are advantageously added one or more active principles to give polymer/CD/drug ternary mixtures.

The release system according to the invention is obtained by means of a process the reaction conditions of which do not entail a chemical alteration of the polymer(s) used and the polymer keeps its mucoadhesive properties essentially unaltered with respect to its original starting properties, since the added CD does not chemically modifies its features. More in particular, during the preparation of the system of the invention, the conditions are chosen so that the crosslinking rate of the polymer is not modified. In addition, the role of the CD within the polymer is not only to help with the coordination of the active principle, but also to act as an agent that, by lowering the polymer crystallinity, makes the drug distribution within the polymer matrix uniform.

Another object of the present invention are release systems in the form of bioadhesive polymer films comprising a cyclodextrin (CD) combination and active principles according to the herein enclosed claims.

Another object is the process by which said films are obtained.

Still another object is the use of said films for locally acting on epithelial wet surfaces, in particular in mucous tissues, more in particular in the pathologies of the buccal cavity.

Further objects will be evident from the following detailed description of the invention.

### Brief Description of the Drawings

Figures 1A and 1B are scanning electron microscopy images (SEM) (x1000 enlargment) of a PEO film (Fig. 1A) and of PEO/HPβCD with 54% HPβCD as prepared in Example 1 (Figure 1B) (inserts show higher enlargments);
Figures 2A and 2B are scanning electron microscopy images (x1000 enlargment) of the PEO film containing triamcinolone acetonide (TrA) (Fig. 2A) or of the PEO/HPβCD film containing TrA prepared according to Example 2 (Fig. 2B) (inserts show higher enlargments);
Figure 3 shows the fusion enthalpy of the PEO/HPβCD films containing different percentages of CD;
Figure 4 shows the enthalpy and fusion temperature of the PEO and PEO/HPβCD film, with and without TrA;
Figure 5 illustrates the relationship between the amount of TrA and the weight of a film portion. On the left: PEO film. On the right: PEO/HPβCD film;
Figures 6 and 7 show the release profiles of TrA from PEO films (lower curve) and PEO/HPβCD films (upper curve) in simulated saliva. Figures 6A and 6B show release profiles of TrA from PEO films (lower curve) and PEO/HPβCD films (higher curve) in simulated saliva. Fig. 6A: a 44.2 cm² film. Fig. 6B: a 0.5 cm² film;
Figures 7A and 7B and 7C refer to: (7A) Raman image of the composition of Example 2 without CD (7B and 7C). Inserts show the spectra acquired at the indicated positions. The Raman image was elaborated by taking into account the intensity of TrA peak at 167 cm⁻¹, it confirms that the matrix (PEO) without CD produces aggregates of non-solubilized TrA in the matrix;
Figures 8A and 8B refer to: (8A) Raman image of the TrA/CD/PEO mixture composed as in Example 2 (8B) The insert shows the spectra acquired at the indicated positions. The Raman image was elaborated taking into account the intensity ratio I₁₃₂₀/I₃₆₂ which indicate CD and PEO, respectively;
Figures 9A and 9B refer to: (9A): Raman image of the TrA/CD/PEO mixture with the composition of Example 2. (9B) The insert shows the spectra acquired at the the indicated positions. The Raman image was elaborated taking into account the intensity ratio I₁₆₆₈/I₃₆₈ that indicates TrA and PEO, respectively.

### Detailed Description of the Invention

According to the invention, the wording: ternary mixture of polymer/CD/drug refers to a polymer or a copolymer or mixtures of polymers and copolimers, the acronym CD refers to cyclodextrins, alone or in a mixture thereof, which are used in the pharmaceutical and cosmetic fields, the wording "drug" indicates one or more active principles, alone or in a mixture thereof, commonly used in the pharmaceutical, nutraceutical and cosmetic fields.

The process for obtaining the release systems according to the invention comprises the basic steps of:
(i) preparing a mixture containing the bioadhesive polymer and the cyclodextrin in water, so that an homogeneous dispersion free from air bubbles is obtained;
(ii) subsequently adding an organic polar aprotic hydrosoluble solvent, such as acetone or ethyl alcohol and mixtures thereof;
(iii) pouring the mixture thus obtained onto a plastic support and let it dry to obtain a film of the desidered thickness.

In steps (i) and/or (ii) one or more active principles can be added as additional components of the polymer/cyclodextrin mixture, choosing the operative conditions, above all in terms of concentration and solvent, so that a complete solubilization of the active principles is obtained.

The steps from (i) to (iii) are carried out in conditions which do not allow any chemical polymerization between reagents, but only creation of labile and transient interactions, in addition to being reversible, of the hydrogen type, electrostatic and Van der Waals interactions.

In this way, the bioadhesive features of the biopolymer are not modified.

According to the invention, the mixtures obtained by the described process (polymer/CD and polymer/CD/active principle) are solutions under all respects, as they are homogeneous (the chemical composition is substantially the same in every point) and the single components form a molecular dispersion in just one physical phase, which, in this case, is similar to a solid solution.

Tipically, the stirring can be carried out at room temperature, in conditions of continuous and moderate agitation for time periods of less than 5 hours. Preferably, the percentage of CD in weight in the film ranges between 10 and 70%, more preferably around 50%. The polymer concentration in the aqueous phase is preferably comprised between 0.26 and 0,5% w/v and the polymers and copolymers that can be used are polyethylene oxides, polyacrilic acids, polyvinyl alcohols, cellulose semi-synthetic derivatives, hydrophilic derivatives of starch, alginates, pectins, chitosans, polysaccharides, natural gums, more preferably polyethylene oxides with a molecular weight higher than 600 kDa, most preferably in a mixture containing an equal amount in weight of polyethylene oxide having a molecular weight higher than 4000 kDa. Also, different polymers and copolymers can be mixed together.

The cyclodextrins used are preferably semi-synthetic derivatives of the β-CD which are soluble in highly polar organic solvents, preferably semi-synthetic cyclodextrins such as HPβCD and methylβ-CD already described above.

Taste correctors can be added to the mixture polymer/dextrin, for example essential oils, natural or synthetic flavourings, sweeteners, colours approved for food and pharmaceutical use. These compounds can have more than one role and therefore their function in the system can be different from the standard classification. In the case of volatile substances, these can be first adsorbed onto the CD by mixing.

Hydrosoluble, pharmacologically active substances can first be dissolved in water and subsequently added, or can be directly added to the mixture polymer/cyclodextrin of the stage (i).

Liposoluble, pharmacologically active substances can first be dissolved in the organic solvent used in stage (ii) and can be added to the polymer/cyclodextrin mixture.

A number of active principles with different pharmacological activities can also be solubilized. According to the present invention, with the term active principle or active compound are meant both the pharmacologically active compounds and the compounds which are endowed with beneficial properties for the body, such as cosmetic compounds or essential oils or nutraceutics.

The active principles can also be of herbal origins, for example essential oils, tinctures and fluid extracts. The active principles, alone or in mixture thereof, are preferably substances that belong to the class of antibiotics, antimicrobial drugs, steroidal and non-steroidal antinflammatory drugs, antioxidants, antivirals, local anesthetics, antimicotic drugs, anticoagulants, wound-healing drugs, cytostatics, bone stimulants, epithelialization drugs.

The ratio active substance/solvent preferably is below 5% (w/v). Typically, the solvents that can be used are methanol, ethanol, acetone; the preferred solvents are ethanol and acetone, particularly preferred is ethanol. Liposoluble flavouring substances can be added to the drug solution, for example essential oils.

The polymer/cyclodextrin dispersion and the organic solvent are mixed in a ratio comprised between 1:3 to 1:100. Preferably, the organic solvent is added to the polymer and dextrin mixture. Tipically, the solution is homogeneous after 4 hours of moderate mixing at room temperature.

The mixture containing polymer, CD and active substance or drug is poured onto a plastic support (polyethylene, polyethyleneterephtalate), preferably a polyethylene support of adequate dimensions, it preferably has a density in between 30 and 150 g/m², more preferably 60 g/m². Drying is carried out at temperatures comprised between 25 and 80°C, preferably at room temperature.

By means of the process acoording to the present invention films of a thickness of 0.01-2 mm can be obtained, preferably 0.015-0.2 mm, wherein the drug is dispersed in a molecular form, which are endowed with a high flexibility and which adapt very well to the mucosa, in particular the buccal mucosa.

The film can be divided in smaller units, typically 10 x 10 cm, and packed in an adequate packaging for pharmaceutical purposes. Each unit can be further cut with scissors at the time of application to adapt the film dimensions to the mucosal lesion, in particular buccal mucosal lesions. It can be manufactured in strips and wound to make a ready-to-use band. Advantageously, all the single smaller fractions have in pratice the same active product concentration.

The salient features of the bioadhesive films of the invention reside in the chemical physical features that describe them.

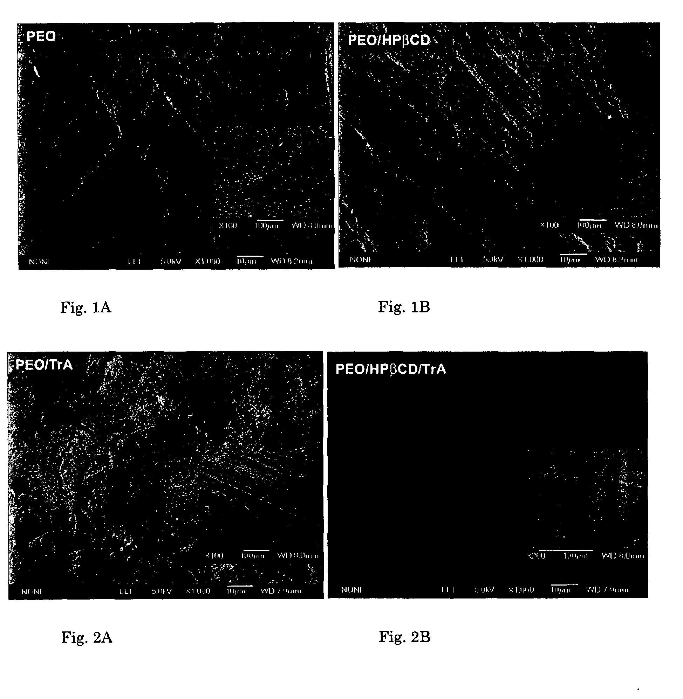

In the first place, the electron scanning microscope analysis of PEO films (Fig. 1A) clearly shows that the surface of the films without HPβCD is not uniform, while films containing HPβCD (Fig. 1B) show a more homogeneous structure.

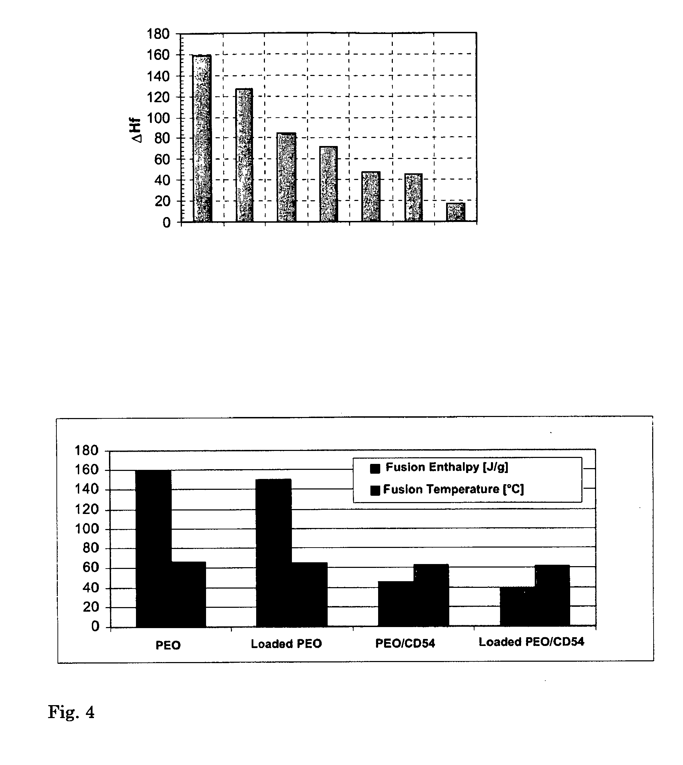

Secondly, the calorimetric analysis shows that PEO/HPβCD films are characterized by a less marked crystallinity with respect to the PEO therein comprised, thanks to the plasticizing action of HPβCD (Fig. 4). As shown in Fig. 3, the effect of CD on the fusion enthalpy depends on the amount found in the film, but amounts as small as 10% already allow a lowering of 20% of the crystallinity both in the presence and in the absence of the active principle. In this way, the CD provides an advantageous flexibility to the polymer film, which thing allows raising the number of amorphous domains which are capable of housing the drug.

In addition to this, the mucoadhesive features of the film, as tested in healthy subjects, are not influenced in a negative way by the presence of CD, on the contrary they are kept practically unaltered. As a matter of fact, PEO/HPβCD films disappeared in about 15 minutes from the application onto the buccal mucosa. No significant difference was seen between men and women.

A further salient feature of the bioadhesive films of the invention containing PEO/HPβCD consists in its good ability to trap and release active principles which are poorly soluble in water.

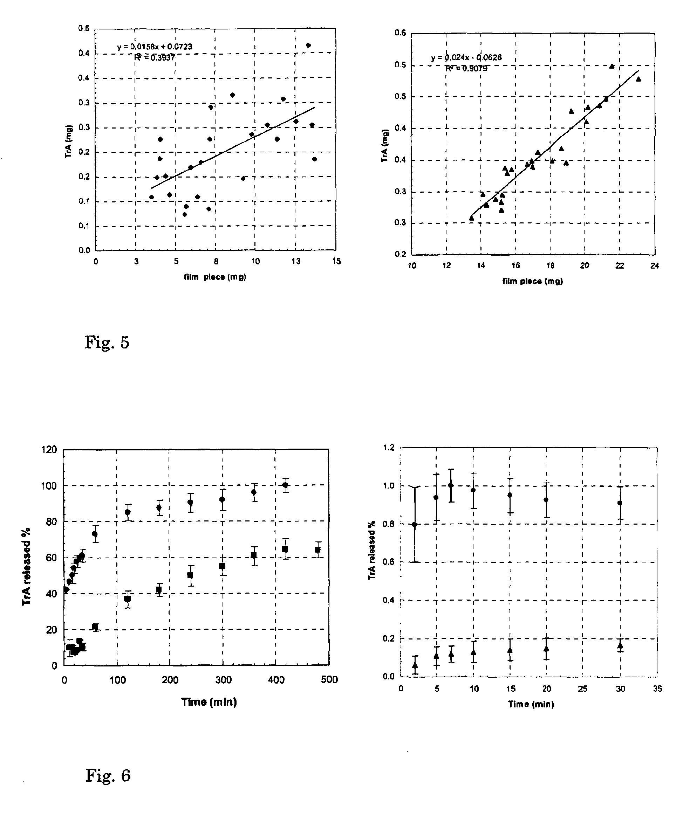

Such a capability was tested for the buccal release of triamcinolone acetonide (TrA), a corticosteroid drug poorly soluble in water. According to the European Pharmacopeia, TrA is a drug that is practically not soluble in water, since its solubility in this vehicle is about 0.02 mg/ml. The presence of HPβCD in the film enhances TrA solubility by 600 fold, i.e., to about 4% w/v (molar ratio TrA/HPβCD 1:10). By further raising the HPβCD concentration no further enhancement of TrA solubility is seen. The release kinetics of TrA from the films are illustrated in Fig. 6. As it can be seen, addition of HPβCD enhances the release rate of the drug and allows a complete release of TrA within 400 minutes. A similar performance was also seen for portions of smaller dimensions, even though the release time was smaller because of the total surface reduction.

What described with reference to TrA is very different and much more advantageous with respect to the Prior Art teachings. As a matter of fact, the conditions disclosed in the above mentioned patent application US 2007/0292479 and meant for the manufacturing of a bioadhesive film containing drugs poorly soluble in water, such as TrA, provide a preliminary solubilization of the drug in a highly concentrate HPβCD solution. The complete solubilization of TrA, anyway, does not happen in actual facts, since, even when using an HPβCD solution near the solubility limits (35% as indicated in US 2007/0292479), it is not possible to quantitatively solubilize the TrA to subsequently obtain a film corresponding to the compositions disclosed in said Prior Art document. There ensues obtaining non-homogeneous films in terms of drug/polymer ratio and which contain TrA in a precipitated form. On the contrary, the process that is the object of the present invention allows a quantitative solubilization of TrA and obtaining homogeneous films (see Figs. 2A and 2B). This aspect is further evident when the films are radially cut in different portions of different dimensions and the amount of TrA contained in each portion in evaluated. The results are illustrated in Fig. 5 as the amount of TrA vs. the weight of the film portion. As it can be seen, for the PEO film there is no correlation between the weight of the portion and the corresponding TrA amount in weight. On the contrary, in the PEO/HPβCD/TrA films was found a linear correlation, which is suggestive of the fact that the CD allows a homogeneous distribution of the drug at the macroscopic level. This means that the PEO/HPβCD/TrA films not only conform to the specifications of the Pharmacopeia for divisible pharmaceutical forms, but can also be cut and adapted to the dimensions of a lesion possibly present in the mouth keeping a constant distribution of the drug amount.

Addition of the drug does not change the fusion temperature of PEO and the associated fusion enthalpy (Fig.4) shows that the polymer platform crystallinity does not change even in the presence of a third component (drug or active component in general) within the matrix. This aspect is important since an enhancement of the amorphous portion, in addition to providing the film with flexibility, allows a better and more homogeneous distribution of the drug followed by the control of its distribution within the matrix, providing the advantage of obtaining films with a homogeneous composition at an industrial level.

In addition, bioadhesive films of the invention obtained by the described method do not show drug crystallization and allow the partition of large amounts of active principles in the area that is in contact with the mucosa, and the subsequent rapid and quantitative release of the drug at the interface with the mucosa, thus avoiding its swallowing and passing at the systemic level, thus maximizing the active principle partition into the mucosa to obtain the desired pharmacological local effect.

The features described above which refer to the films of the invention allow their dermatologic use. In the surgical frame it can be of great clinical usefulness being capable of modulating the activation of tissue reparation and regeneration processes at the basis of the healing process. In this context, some devices such as, for example, mucoadhesive membranes and/or strips, manufactured with biodegradable material, loaded with pharmacologically active substances, and capable to facilitate the development of the reparation phases following to a surgical intervention, may be very useful. The possibility of a local delivery of a drug by means of biocompatible and absorbable matrixes can allow the topical treatment of a number of diseases of the oral mucosa, also ensuring the advantage of a drastic reduction with respect to the dosage administered by the systemic route and, therefore, of the subsequent side effects. Thus, these systems have a great clinical advantage, above all for those patients in which concomitant liver or renal pathologies make an effective systemic therapy non feasible. The prolonged, in situ release allows reducing the therapeutic regime via the systemic route and a better local control of the inflammatory/bacterial phenomena. This allows the optimization both of the professional clinical intervention and of the pharmacological therapy in patients with poor compliance. The reduced local concentrations of the drug, while keeping their therapeutic effectiveness, may allow the parallel administration of drugs that, if administered by the same route, might give place to pharmacokinetic interferences.

Specifically, the system of the invention may be very helpful in the following cases:
- Treatment of cutaneous lesions with exudate, such as wounds, sores, burns, ulcers;
- Topical treatment of wounds, such as surgical ones, to facilitate their healing and to facilitate tissue regeneration;
- Topical pharmacological treatment (with corticosteroids, anesthetics, antibiotics, antinflammatory drugs) of the surgical wounds of the oral cavity (post-extraction alveolus, sutures with first intention healing, mucous wounds with second intention healing);
- Topical pharmacological treatment of pathological oral diseases (lichen, pemphigus);
- Topical pharmacological treatment of oral diseases such as traumatic ulcers and aphtae;
- Topical pharmacological treatment (clorexidine) of parodontal affections (transmucosal treatment of periodontal pouches).

The film can contain not only pharmacologically active substances but also substances for cosmetic, nutracetic use and micronutrients, in particular to facilitate tissue regeration processes. Thus, the fields of application of the release systems of the invention are the medical, pharmaceutical, cosmetic and cosmeceutical (cosmetic with therapeutic purposes) and nutraceutical fields.

The film can be applied as it is by gently pressing it. It can find applications in humans and in the veterinary field.

The process of the invention allows to:
- avoid using heat to solubilize a drug and/or active principle, which is not advantageous for the economy of the process at the industrial level,
- prepare a transparent drug and/or active principle solution, polymer and CD, which is a good indicator for obtaining a film having an homogeneous composition and complying with the Pharmacopea requisites, and
- prepare a low concentration solution of a bioadhesive polymer with no formation of air bubbles, thanks to the decrease in the viscosity of the solution provided by the polymer/CD interaction.

A fundamental advantage of the process of the invention resides in the fact that the bioadhesion/mucoadhesion starting features of the polymer are not altered when the latter is part of the ternary polymer/CD/active principles, instead they are improved. There exists no standard test for the evaluation of mucoadhesion, due to the different physical forms and of the variability of the biological substrates, anyway a good indication is given by the tests described in the following experimental section, where experiments will be shown to demonstrate what alleged above.

Further advantages reside in the fact that the addition of cyclodextrin does not modify the bioadhesion features of the polymer, which can therefore be chosen as a function of the specific uses and loaded with the desired active principle(s). As a matter of fact, the results of tests carried out with a polymer film loaded with CD evidently show that the addition of CD to the film does not change the adhesion time and the adhesion features to the mucosa.

The following Examples are given to illustrate the invention and should not be considered as limiting to its scope.

### Esempi

Following are illustrated the experimental results obtained with unloaded (with no drug) and TrA-loaded films, containing or not containing CD.

### Example 1

### PEO/CD films containing 54% w/w of HPβCD

157.5 mg of PEO 400 kDa, 157.5 mg of PEO 6000 kDa and 375 mg of HPβCD were solubilized in 90 ml of deionized water under magnetic stirring for 3 hours at room temperature. To the solution were added 30 ml of ethanol under moderate stirring. The clear solution was poured into teflon dishes with a diameter of 37.5 cm and let dry at room temperature until reaching a constant weight. The obtained film had a 0.0017 mm thickness and a density of 32 g/m² and was analyzed by scanning electron microscopy and scanning differential calorimetry (Figs. 1 and 2)

### Example 2

### PEO/CD films containing a practically water-not soluble drugs and in particular TrA.

315 mg of PEO 400 kDa, 315 mg of PEO 6000 kDa and 750 mg of HPβCD were solubilized in 90 ml of deionized water under magnetic stirring for 3 hours at room temperature. 15 mg of TrA were solubilized in 30 ml of a solution of 96° ethanol under moderate stirring per a few minutes. The solution of TrA in ethanol was poured onto the water solution of PEO/HPβCD and left under stirring for 4 hours. The clear solution was poured in teflon dishes with a diameter of 37.5 cm and let dry at room temperature until reaching a constant weight. A film of 0.035 mm of thickness was obtained and a density of 64 g/m² and containing a dosage of 0.07 mg/cm² of TrA.

### Reference Example 2

### PEO films containing: a practically water-not soluble drug and in particular TrA

As a reference sample, the film of Example 2 was made without addition of cyclodextrin.

### Example 3

### PEO/CD films containing a water-soluble drug

157.5 mg of PEO 400 kDa, 157.5 mg of PEO 6000 kDa and 375 mg of HPβCD were solubilized in 90 ml of deionized water under magnetic stirring for 3 hours at room temperature.

1.26 mg of clorexidine were added to the PEO and HPβCD solution to which were also added 30 ml of ethanol under moderate stirring for 4 hours. The solution was poured in teflon dishes with a diameter of 37.5 cm and let dry at room temperature until reaching a constant weight. A film with a 0.017 mm of thickness was obtained and a density of 32 g/m².

### Example 4

### PEO/CD films containing an essential oil

80 mg of peppermint essential oil were adsorbed onto 375 mg of HPβCD by means of stirring. The paste was dispersed in 90 ml of deionized water containing 157.5 mg of PEO400 kDa and 157.5 mg of PEO 6000 kDa under magnetic stirring for 3 hours at room temperature. To the dispersion 30 ml of ethanol were added under moderate magnetic stirring. The solution was poured in teflon dishes with a diameter of 37.5 cm and let dry at room temperature until reaching a constant weight. The film obtained had a 0.017 mm of thickness and a density of di 32 g/m².

### Example 5

### Control of the TrA and CD distribution within the PEO matrix

The Raman spectra were collected by means of a confocal Raman spectrometer model ARAMIS (Horiba-Jobin Yvon). The source of excited light was a photodiode laser which emitted in the visible (green) at 532 nm. The backscattered diffraction at 180° was gathered by an Olympus MPlan 50x, NA = 0.75. The confocal opening and the slit on the instrument were both set at 200 µm, and a grating with 1200 grooves/mm was used. The radiation was focused onto a CCD (Charge Coupled Device) detector cooled to -70°C and provided with a Peltier cell.

The Raman mapping with confocal sampling was used to analyze the distribution of TrA within the binary PEO/TrA system (Reference Example 2) and within the ternary PEO/HPβCD/TrA system (Example 2). The Raman image was collected onto a 13 x 13 µm surface

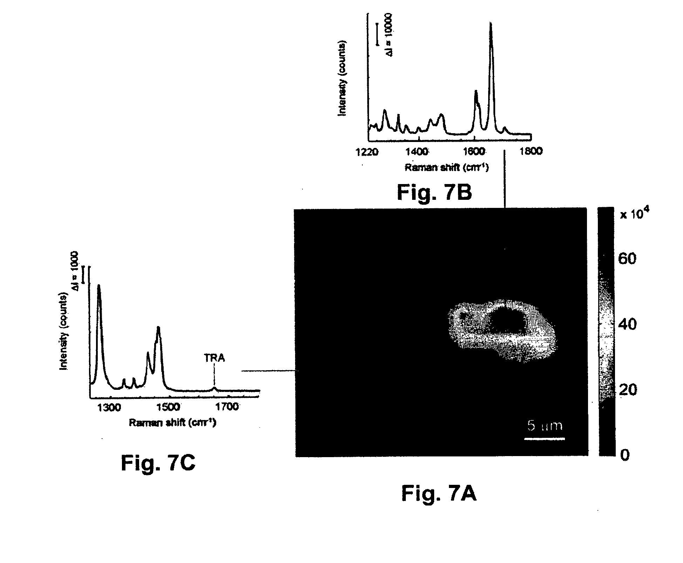

TrA is not soluble in PEO. In the Raman images of Figs. 7A, 7B and 7C, which refer to the composition of the Reference Example 2, the TrA crystals are clearly evidenced. It can also be noticed that the TrA signal is present in those regions where PEO is widely predominant, even though the peak is barely visible. This means that a very small amount of TrA is anyway soluble into PEO, and this fraction can be quantitated by means of confocal Raman spectroscopy. In any case, the spectroscopic data show that the amount of TrA that can be directly solubilized in PEO is extremely small.

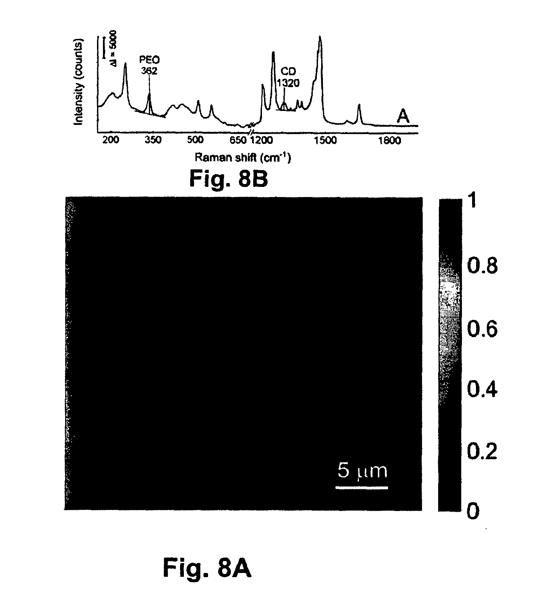

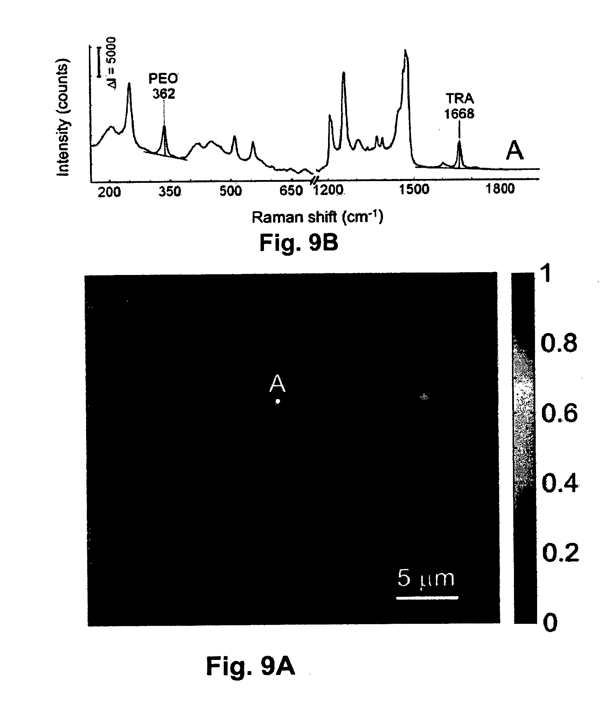

In Figs. 8A and 8B the Raman image of the ternary TrA/HPβCD/PEO mixture having the composition of Example 2. Fig 8A was obtained by considering the intensity ratio of the HPβCD peak at 1320 cm⁻¹ and the PEO one at 362 cm⁻¹. The colour uniformity of the image in Fig. 8B (I1320/I362 = 0.4 ± 0.05) shows a constant value of the molar ratio of the two components, and, thus, a uniform distribution of HPβCD in PEO. A similar feature is shown by the images obtained taking into consideration the TrA peak at 1670 cm⁻¹, normalized with respect to the same PEO peak (Figs. 9A and 9B). A comparison between the Raman images of Fig. 7A-C with respect to Figs. 8B and 9A-B show that the use of HPβCD promotes a uniform dispersion of TrA within PEO, avoiding its precipitation in crystal form, as, on the contrary, it happens in the same conditions but in absence of HPβCD.

The intensity map obtained with Raman and corresponding to Figs. 8 and 9 shows a constant colour, indicative of a homogeneous mixture of PEO and CD and TrA respectively, indirectly showing the homogeneity of the PEO/CD/TrA mixture and thus the molecular dispersion of the three components between them.

### Example 6

### Mucoadhesion Assays of Films

**Table 1**

| **Film** Prepared according to | **Active Principle [3 mg]** | **Polymer [63 mg]** | **Cyclodextrin [75 mg]** | **Detachment from mucosa** |
|---|---|---|---|---|
| Example 1 | - | PEO 6000 kDa | HpβCD | NO |
| Example 2 | TrA | PEO 6000 kDa | HpβCD | NO |
| Example 1 | - | Polyvinylalcohol (Mowiol 40-88; average Mw -205 205 kDa) | HpβCD | NO |
| Example 2 | TrA | Polyvinylalcohol (Mowiol 40-88; average Mw -205 kDa) | HpβCD | NO |
| Example 1 | - | Chitosan (Protasan UPG 213; 400 kDa) | HpβCD | NO |
| Example 2 | TrA | Chitosan (Protasan UPG 213; 400 kDa) | HpβCD | NO |

From each film 2x2 cm samples were obtained

The tests were carried out on pig buccal mucosa fixed to a teflon support. Each sample was applied by adhesion to the mucosa. The whole preparation was placed in a dissolving tank containing 200 ml of simulated saliva (2.38 g Na₂HPO₄, 0.19 g KH₂PO₄, 8 g NaCl per liter, adjusted to pH 6.8 with orthophosphoric acid) under stirring at 37°C and 30rpm.

The behaviour of the film was monitored in terms of adhesion and erosion. The results are shown in Table 1

## Claims

1. A process for producing a release system comprising a bioadhesive polymer or polymer mixture and a percentage of cyclodextrin in weight with respect to the polymer which ranges from 10 to 70%, preferably about 50%, comprising the stages of:
(i) preparing a mixture containing the polymer and the cyclodextrin in water, at room temperature;
(ii) adding an aliquot of an organic polar aprotic hydrosoluble solvent, such as acetone or ethyl alcohol and mixtures thereof, in a ratio ranging from 1:3 to 1:100;
(iii) pouring the mixture obtained in stage (ii) onto a plastic support and let dry at a temperature ranging from 25 to 80°C, preferably at room temperature, to obtain a film of the desired thickness;
the steps from (i) to (iii) being carried out in conditions which do not allow any chemical polymerization between reagents.

2. The process according to claim 1, wherein, to the polymer/dextrin mixture one or more hydrosoluble active compound(s) and /or one or more liposoluble active compound(s), the latter two dissolved beforehand in the organic solvent used in step (ii), and/or one or more volatile compounds after having being absorbed onto the cyclodextrin, are added.

3. A release system obtained according to any one of claims 1-2, **characterized by** the fact that the cyclodextrin forms a molecular dispersion with the polymer or polymer mixture and shows a decrease in the crystallinity measured as fusion enthalpy, at least 20% lower with respect to the polymer or polymer mixture alone.

4. The release system according to claim 3, wherein the polymer is chosen from among polyacrylic acids, polyvinyl alcohol, cellulose semi-synthetic derivatives, starch hydrophilic derivatives, alginates, pectine, chitosan, polysaccharides, natural gums, polyethylene oxides, preferably polyethylene oxides having a molecular weight higher than 600 kDa, more preferably in a 50% mixture with a polyethylene oxide having a molecular weight higher than 4000 kDa, and mixtures therof.

5. The release system according to claims 3-4, wherein the cyclodextrins are β-cyclodextrin and derivatives thereof, preferably HP-beta-CD and methyl-beta-CD.

6. The release system according to claims 3-5, further comprising an active principle chosen from among antibiotics, antimicrobial drugs, steroidal and non-steroidal anti-inflammatory drugs, antioxidants, antivirals, local anesthetics, antimycotic drugs, anticoagulants, wound-healing drugs,
cytostatics, bone stimulants, epithelialization drugs, essential oils, flavourings, sweeteners, colours, substances for cosmetic and nutraceutic use, and micronutrients, and mixtures thereof, in particular triamcinolone acetonide and chlorhexidine.

7. The release system according to claims 3-6, in the form of a bioadhesive film comprising a concentration of active principle comprised in between 0.01 mg/cm² and 1 mg/cm², being the film preferably combined to a plastic support.

8. The release system according to claims 3-7, for use in the local treatment of the regeneration and reparation processes of tissues and of the mucosal diseases in animals and in humans.

9. The release system for use according to claim 8, wherein the tissue regeneration processes relates to healing of wounds, sores, burns, ulcers.

10. The release system for use according to claim 8, wherein the mucosal diseases are chosen from among surgical wounds of the oral cavity, oral displays of systemic pathologies, such as lichen, pemphigus, traumatic buccal ulcers, aphtae, periodontal diseases, oral manifestations of systemic pathologies.

11. Manufactured goods, in particular in the form of preparations for occlusive applications, gels, hydrogels, films, patches, comprising the release system according to claims 3-7.

## Patentansprüche

1. Verfahren zur Herstellung eines Freisetzungssystems, umfassend ein bioadhäsives Polymer oder Polymergemisch und einen Gewichtsprozentsatz an Cyclodextrin bezogen auf das Polymer, der von 10 bis 70 % reicht, bevorzugt etwa 50 % beträgt, umfassend die Stufen:
(i) Herstellen eines Gemisches, enthaltend das Polymer und das Cyclodextrin, in Wasser bei Raumtemperatur;
(ii) Zugeben eines Aliquots eines organischen polaren aprotischen wasserlöslichen Lösungsmittels, wie Aceton oder Ethylalkohol und Gemische davon, in einem Verhältnis im Bereich von 1 : 3 bis 1 : 100;
(iii) Gießen des in Stufe (ii) erhaltenen Gemisches auf einen Kunststoffträger und Trocknenlassen bei einer Temperatur im Bereich von 25 bis 80 °C, bevorzugt bei Raumtemperatur, unter Erhalt eines Films mit der gewünschten Dicke;
wobei die Schritte (i) bis (iii) unter Bedingungen durchgeführt werden, die keine chemische Polymerisation zwischen den Reagenzien zulassen.

2. Verfahren nach Anspruch 1, wobei dem Polymer/Dextrin-Gemisch eine oder mehrere wasserlösliche aktive Verbindung(en) und/oder eine oder mehrere fettlösliche aktive Verbindung(en), wobei letztere zuvor in dem in Schritt (ii) verwendeten organischen Lösungsmittel gelöst werden, und/oder eine oder mehrere flüchtige Verbindung(en), nachdem sie auf dem Cyclodextrin absorbiert worden sind, zugegeben wird/werden.

3. Freisetzungssystem, erhalten nach einem der Ansprüche 1 - 2, **dadurch gekennzeichnet, dass** das Cyclodextrin eine molekulare Dispersion mit dem Polymer oder Polymergemisch bildet und eine verringerte Kristallinität zeigt, gemessen als Schmelzenthalpie, die, bezogen auf das Polymer oder Polymergemisch allein, mindestens 20 % geringer ist.

4. Freisetzungssystem nach Anspruch 3, wobei das Polymer ausgewählt ist aus Polyacrylsäuren, Polyvinylalkohol, semi-synthetischen Cellulosederivaten, hydrophilen Stärkederivaten, Alginaten, Pektin, Chitosan, Polysacchariden, natürlichen Kautschuken, Polyethylenoxiden, bevorzugt Polyethylenoxiden mit einem Molekulargewicht von mehr als 600 kDa, stärker bevorzugt in einem 50%igen Gemisch mit einem Polyethylenoxid mit einem Molekulargewicht von mehr als 4000 kDa, und Gemischen davon.

5. Freisetzungssystem nach den Ansprüchen 3 - 4, wobei die Cyclodextrine β-Cyclodextrin und Derivate davon, bevorzugt HP-beta-CD und Methyl-beta-CD sind.

6. Freisetzungssystem nach den Ansprüchen 3 - 5, ferner umfassend einen aktiven Grundbestandteil, ausgewählt aus Antibiotika, antimikrobiellen Arzneimitteln, steroidalen und nichtsteroidalen entzündungshemmenden Arzneimitteln, Antioxidationsmitteln, Virostatika, lokalen Anästhetika, Antimykotika, Antikoagulanzien, Wundheilungsmitteln, Zytostatika, Knochenstimulanzien, Arzneimittel für die Epithelialisierung, ätherischen Ölen, Aromastoffen, Süßungsmitteln, Färbemitteln, Substanzen zur kosmetischen und nutraceutischen Verwendung und Mikronährstoffen und Gemischen davon, insbesondere Triamcinolonacetonid und Chlorhexidin.

7. Freisetzungssystem nach den Ansprüchen 3 - 6 in Form eines bioadhäsiven Films, umfassend eine Konzentration des aktiven Grundbestandteils, die zwischen 0,01 mg/cm² und 1 mglcm² liegt, wobei der Film bevorzugt mit einem Kunststoffträger kombiniert wird.

8. Freisetzungssystem nach den Ansprüchen 3 - 7 zur Verwendung bei der lokalen Behandlung der Regenerations- und Wiederherstellungsprozesse von Geweben und der Schleimhauterkrankung bei Tieren und Menschen.

9. Freisetzungssystem zur Verwendung nach Anspruch 8, wobei sich die Geweberegenerationsprozesse auf die Heilung von Wunden, Geschwüren, Verbrennungen, Ulzera beziehen.

10. Freisetzungssystem zur Verwendung nach Anspruch 8, wobei die Schleimhauterkrankungen ausgewählt sind aus chirurgischen Wunden der Mundhöhle, oralen Anzeichen systemischer Pathologien, wie Flechte, Pemphigus, traumatischen Wangengeschwüren, Aphten, Paradontose, oralen Manifestationen systemischer Pathologien.

11. Industriegüter, insbesondere in Form von Präparaten für okklusive Anwendungen, Gele, Hydrogele, Filme, Pflaster, umfassend das Freisetzungssystem nach den Ansprüchen 3 - 7.

## Revendications

1. Procédé pour produire un système de libération comprenant un polymère bio-adhésif ou un mélange de polymères et un pourcentage de cyclodextrine en poids par rapport au polymère qui varie de 10 à 70%, préférentiellement environ 50%, comprenant les étapes de :
(i) préparation d'un mélange contenant le polymère et la cyclodextrine dans l'eau, à température ambiante ;
(ii) addition d'un aliquot d'un solvant organique hydrosoluble aprotique polaire, tel que l'acétone ou l'alcool éthylique et leurs mélanges, dans un rapport allant de 1 :3 à 1 :100 ;
(iii) dépôt du mélange obtenu à l'étape (ii) dans un support en plastique et séchage à une température allant de 25 à 80°C, préférentiellement à température ambiante, pour obtenir un film à l'épaisseur désirée ;
Les étapes de (i) à (iii) étant réalisées dans des conditions qui ne permettent pas de polymérisation chimique entre les réactifs.

2. Procédé selon la revendication 1, dans lequel, sont ajoutés au mélange polymère/dextrine un ou plusieurs composé(s) actif(s) hydrosoluble(s) et/ou un ou plusieurs composé(s) actif(s) liposoluble(s), les deux derniers préalablement dissous dans le solvant organique utilisé à l'étape (ii), et/ou un ou plusieurs composés volatiles après avoir été absorbés dans la cyclodextrine.

3. Système de libération obtenu selon l'une quelconque des revendications 1-2, **caractérisé par le fait que** la cyclodextrine forme une dispersion moléculaire avec le polymère ou le mélange de polymères et présente une diminution de la cristallinité mesurée par l'enthalpie de fusion, d'au moins 20% inférieure par rapport au polymère, ou au mélange de polymère seul.

4. Système de libération selon la revendication 3, dans lequel le polymère est choisi parmi les acides polyacryliques, l'alcool polyvinylique, les dérivés de cellulose semi-synthétiques, les dérivés hydrophiles de l'amidon, les alginates, la pectine, le chitosane, les polysaccharides, les gommes naturelles, les oxydes de polyéthylène, préférentiellement les oxydes de polyéthylène possédant un poids moléculaire supérieur à 600 kDa, plus préférentiellement dans un mélange à 50% avec un oxyde de polyéthylène possédant un poids moléculaire supérieur à 4000Kda, et leurs mélanges.

5. Système de libération selon les revendications 3-4, dans lequel les cyclodextrines sont des β-cyclodextrines et leurs dérivés, préférentiellement HP-béta-CD et methyl-beta-CD.

6. Système de libération selon les revendications 3-5, comprenant en outre un principe actif choisi parmi les antibiotiques, les médicaments antimicrobiens, les médicaments anti-inflammatoires stéroïdiens et non stéroïdiens, les antioxydants, les antiviraux, les anesthésiques locaux, les médicaments antimycosiques, les anticoagulants, les médicaments pour la cicatrisation des blessures, les cytostatiques, les stimulants des os, les médicaments favorisant l'épithélisation, les huiles essentielles, les arômes, les édulcorants, les colorants, les substances à utilisation cosmétique et nutraceutique, et les micronutriments, leurs mélanges, en particulier le triamcinolone acétonide, et la chorhexidine.

7. Système de libération selon les revendications 3-6, sous la forme d'un film bio-adhésif comprenant une concentration en principe actif comprise entre 0.01mg/cm² et 1mg/cm², le film étant préférentiellement combiné à un support en plastique.

8. Système de libération selon les revendications 3-7, pour l'utilisation dans un traitement local de régénération et des processus de réparation des tissus et des maladies des muqueuses chez les animaux et les humains.

9. Système de libération selon la revendication 8, dans lequel les processus de régénération des tissus concernent la guérison des blessures, des plaies, des brûlures, des ulcères.

10. Système de libération selon la revendication 8, dans lequel les maladies de la muqueuses sont choisies parmi les blessures chirurgicales de la cavité orale, les manifestations orales de pathologies systémiques, telles que le lichen, le pemphigus, les ulcères buccaux traumatiques, les aphtes, les maladies parodontales, les manifestations orales de maladies systémiques.

11. Produits manufacturés, en particulier sous la forme de préparations pour des applications occlusives, des gels, des hydrogels, des patchs, comprenant le système de libération selon les revendications 3-7.
